# EUROPÄISCHE PATENTANMELDUNG

(11) **EP 4 847 600 A2**
(43) Veröffentlichungstag der Anmeldung: **02.12.2026**
(21) Anmeldenummer: 26181650.8
(22) Anmeldetag: 28.05.2026
(51) Int. Cl.: C12N 1/14, C12M 1/00

(54) **VERFAHREN UND PRODUKTIONSANLAGE ZUR HERSTELLUNG VON MYZELWERKSTOFF**

(30) Priorität: 28.05.2025 DE 102025120929
(71) Anmelder: Fraunhofer-Gesellschaft zur Förderung der angewandten Forschung e.V., 80686 München (DE)
(72) Erfinder: Eiting, Melanie, 46047 Oberhausen (DE); Wack, Holger, 46047 Oberhausen (DE); Vieres, Lina, 46047 Oberhausen (DE); Nühlen, Jochen, 46047 Oberhausen (DE)
(74) Vertreter: Von Rohr Patentanwälte Partnerschaft mbB

(57) **Zusammenfassung**

Dargestellt und beschrieben ist ein Verfahren zur Herstellung von mit Pilzmyzel durchwachsenem Substratmaterial als substratbasierter Myzelwerkstoff (1), welches die Verfahrensschritte Bereitstellung eines Substratmaterials als Grundsubstrat (3) für die Herstellung von substratbasiertem Myzelwerkstoff (1), Inokulation des Substratmaterials mit einem Inokolum (5), Durchwachsen des Substratmaterials mit Pilzmyzel in einer gegenüber der Umgebungsatmosphäre zumindest im Wesentlichen geschlossenen und/oder abtrennbaren Einhausung (4) während einer Durchwachstumsphase des Substratmaterials zur Bildung von substratbasiertem Myzelwerkstoff (1) in der Einhausung (4) und Ausbringung von durchwachsenem Substratmaterial als Myzelwerkstoff (1) aus der Einhausung (4) vor einer Fruchtkörperbildung aufweist, wobei Substratmaterial kontinuierlich in die Einhausung (4) eingebracht und/oder durchwachsenes Substratmaterial kontinuierlich aus der Einhausung (4) ausgebracht wird und/oder wobei Substratmaterial innerhalb der Einhausung (4) kontinuierlich von einem Einbringungsort (19) des Substratmaterials als Grundsubstrat (3) in die Einhausung (4) zu einem Ausbringungsort (20) des Substratmaterials als Myzelwerkstoff (1) aus der Einhausung (4) transportiert wird.

## Beschreibung

Die vorliegende Erfindung betrifft ein Verfahren und eine Produktionsanlage zur Herstellung von mit Pilzmyzel durchwachsenem Substratmaterial, das einen substratbasierten Myzelwerkstoff darstellt.

Das technische Anwendungsgebiet der Erfindung liegt in der Werkstofftechnik. Zur Herstellung substratbasierter Myzelwerkstoffe wird Pilzmyzel von Pilzen genutzt, um beispielsweise organische Abfälle, Nebenprodukte und Reststoffe in innovative und nachhaltige Materialien umzuwandeln. Die Schlüsseltechnologie bildet in diesem Zusammenhang das Pilzmyzel. Hierbei handelt es sich um einen spezifischen Bestandteil des Pilzes, der für die Herstellung substratbasierter Myzelwerkstoffe genutzt wird.

Die Erfindung betrifft damit abweichend zu Verfahren und Anlagen zur Herstellung von Speisepilzen, bei denen es um die Fruchtkörperbildung geht, die Herstellung von Werkstoffen, die mit Pilzmyzel durchwachsen sind. Hierbei wird ein möglichst vollständiges Durchwachsen des Werkstoffs mit Pilzmyzel angestrebt, während eine Fruchtkörperbildung unerwünscht ist.

Myzelwerkstoffe oder auch Pilzwerkstoffe eröffnen beispielsweise in Bereichen der Verpackungsindustrie, dem Bauwesen und der Möbelindustrie nachhaltige Alternativen zu bisher etablierten Werkstoffen. Myzelwerkstoffe sind umweltfreundlich, biologisch abbaubar, können auf Basis von Reststoffen der Agrarindustrie hergestellt werden und dazu beitragen, beispielsweise den Einsatz von erdölbasierten Werkstoffen zu reduzieren.

Bei der Herstellung substratbasierter Werkstoffe wird ein Substrat mit Pilzmyzel durchwachsen. Das Myzel bildet ein fadenförmiges Netzwerk und ist oft als sichtbare Schicht auf dem Substrat erkennbar. Diese Art von Werkstoffen hat geringe Dichten und das Myzel fungiert als natürliches Bindemittel. Es entstehen leichte Werkstoffe, bei denen das Myzel eine strukturelle Funktion übernimmt. Wahlweise kann das durchwachsene Substrat unter hohem Druck und Hitze verpresst werden. Dadurch wird das Myzel nicht mehr sichtbar und es entsteht ein Material, das an Pressspanplatten erinnert. Durch die Hitze wird das Myzel zu einem Bioharz, wodurch ein sehr stabiler Werkstoff entsteht.

Die allgemeine Methodik der bisher etablierten Herstellungsprozesse ist in allen Verfahren ähnlich und konzentriert sich auf die Verwendung ausgewählter Pilz-Reinkulturen und eines Nährsubstrats, das vom Pilz verstoffwechselt werden kann. Hierbei werden bei einem bekannten Batch-Prozess ein Grundsubstrat, gebildet aus einem Substratmaterial und einem Nährsubstrat, und ein Pilzmyzel in Formen eingebracht, in der der Pilz in einer kontrollierten Umgebung zu wachsen beginnt. Während des Wachstumsprozesses bildet der Pilz Hyphen, die ein Netz aus Myzel durch und um das Material herum bilden und es dadurch zu einem selbsttragenden Myzelverbund verbinden. Dieser Myzelverbund wird dann aus der Form genommen und vollständig getrocknet, um weiteres Wachstum zu verhindern, so dass ein fertiges Endprodukt erhalten wird.

Zur Trennung des Wachstumsprozesses in zwei separate Wachstumsphasen ist bei einem auf die Anmelderin zurückgehenden bekannten Batch-Prozess vorgesehen, eine Mischung aus Grundsubstrat und Pilzmyzel in Beutel einzubringen, worauf in dem Beutel ein erstes Durchwachsen des Substrats zu einem Myzelverbund erfolgt. Dieser Myzelverbund bildet eine Art "Halbzeug", welches anschließend weiterverarbeitet werden kann. Beispielsweise kann der Myzelverbund aus mehreren Beuteln gesammelt und dann zur Herstellung größerer Bauteile verwendet werden. Da der Myzelverbund bei der Entnahme aus dem Beutel nach der ersten Wachstumsphase in Teilen zerstört wird, erfolgt eine zweite Wachstumsphase in einer Form, in der sich die Hyphen in der endgültigen Form verfestigen. Im Anschluss folgen auch hier die Trocknung und Entformung zum Erhalt des Endprodukts.

Die bislang bekannten Batch-Prozesse zur Herstellung substratbasierter Myzelwerkstoffe sind stark manufakturartig und durch zahlreiche händische Arbeitsschritte geprägt, die eine kontrollierte Massenfertigung und eine Anwendung etwa in der Bauindustrie bislang unmöglich machen. Durch kleinteilige Batch-Prozesse ist ein Scale-Up schwierig. Für ein Scale-Up bekannter formgebundener Verfahren ist eine höhere Anzahl von Formen erforderlich, in die Grundsubstrat und Pilzmyzel eingebracht werden, oder es müssen größere Formen eingesetzt werden. Hier wird versucht, ein Scale-Up durch eine Vergrößerung der Lagerkapazitäten zu erreichen. Daraus resultiert ein hoher Platzbedarf für die Produktion. Beim Wachstum des Pilzes in Formen lassen sich zudem definierte Wachstumsbedingungen nicht oder nur mit großem Aufwand realisieren. Erfolgt das Durchwachsen des Substrats zu einem Myzelverbund in Beuteln, ist die Effizienz der Inkubationen begrenzt, da die Inkubationsbeutel konstruktionsbedingt nur eine kleine Filterfläche zum Gasaustausch besitzen.

Im Übrigen findet die Inokulation bei den bekannten Batch-Prozessen zur Herstellung von mit Pilzmyzel durchwachsenem Substratmaterial eher punktuell statt, was zu einem inhomogenen Durchwachsen des Substratmaterials und einer inhomogenen Struktur des Myzelwerkstoffs führen kann. Durch die in bekannten Batch-Prozessen eingesetzten Einweg-Gefäße oder Beutel, welche meist aus Kunststoff bestehen, entsteht eine große Abfallmenge.

Aufgabe der vorliegenden Erfindung ist es, ein gegenüber den bekannten Batch-Prozessen vorteilhaftes Verfahren und eine Produktionsanlage zur Herstellung von substratbasierten Myzelwerkstoffen zur Verfügung zu stellen.

Insbesondere sollen das zur Verfügung gestellte Verfahren und die zur Verfügung gestellte Produktionsanlage ein hohes Potenzial für eine Skalierung der Technologie aufweisen, eine ökonomische Massenproduktion von Myzelwerkstoff zulassen und damit industrielle Anwendungsbereiche für substratbasierte Myzelwerkstoffe erschließen.

Die vorgenannten Aufgaben werden durch ein Verfahren mit den Merkmalen von Anspruch 1 und durch eine Produktionsanlage mit den Merkmalen von Anspruch 8 gelöst. Vorteilhafte Aspekte, Eigenschaften und Merkmale der vorliegenden Erfindung sind Gegenstand der abhängigen Ansprüche.

Das erfindungsgemäße Herstellungsverfahren sieht die nachfolgenden Verfahrensschritte vor:
- Bereitstellung eines Substratmaterials als Grundsubstrat für die Herstellung von substratbasiertem Myzelwerkstoff und, vorzugsweise, Aufbereitung des Substratmaterials durch Zerkleinerung und/oder Homogenisierung und/oder Befeuchtung und/oder Sterilisation und/oder durch Zugabe von wenigstens einem von wenigstens einem vorbestimmten Pilz verstoffwechselbaren Nährstoff zum Substratmaterial,
- Inokulation des Substratmaterials mit einem mit dem wenigstens einen vorbestimmten Pilz gebildeten Inokolum,
- Durchwachsen des Substratmaterials mit Pilzmyzel in einer gegenüber der Umgebungsatmosphäre zumindest im Wesentlichen geschlossenen und/oder abtrennbaren Einhausung während einer Durchwachstumsphase des Substratmaterials zur Bildung von substratbasiertem Myzelwerkstoff in der Einhausung und
- Ausbringung von durchwachsenem Substratmaterial als Myzelwerkstoff aus der Einhausung vor einer Fruchtkörperbildung und, insbesondere, bei Erreichen eines definierten Durchwachsungsgrades und/oder einer definierten Durchwachstumsdauer des Substratmaterials in der Einhausung.

Zur Lösung der gestellten Aufgaben ist erfindungsgemäß weiter vorgesehen, dass
- Substratmaterial als Grundsubstrat kontinuierlich in die Einhausung eingebracht und/oder durchwachsenes Substratmaterial als Myzelwerkstoff kontinuierlich aus der Einhausung ausgebracht wird und/oder
- dass Substratmaterial innerhalb der Einhausung kontinuierlich von einem Einbringungsort des Substratmaterials als Grundsubstrat in die Einhausung zu einem Ausbringungsort des Substratmaterials als Myzelwerkstoff aus der Einhausung transportiert wird.

Erfindungsgemäß wird erstmalig und in Abweichung von den aus dem Stand der Technik bekannten Batch-Prozessen zur Herstellung von Myzelwerkstoffen eine kontinuierliche Herstellung substratbasierter Myzelwerkstoffe vorgeschlagen. Die bereitgestellte Menge des Grundsubstrats wird kontinuierlich in einem Durchwachstumsprozess in Myzelwerkstoff umgewandelt, der dann als Halbzeug bzw. Zwischenprodukt für eine Weiterverarbeitung in einem anschließenden Verfahrensschritt und/oder für eine Vorratshaltung oder als Handelsprodukt bereitgestellt werden kann.

Die erfindungsgemäße Produktionsanlage weist eine gegenüber der Umgebung zumindest im Wesentlichen geschlossene und/oder abtrennbare Einhausung auf, in der das Myzelwachstum stattfindet. In der Einhausung lassen sich in einfacher Weise definierte Wachstumsbedingungen für das Durchwachsen eines Substratmaterials als Grundsubstrat mit Pilzmyzel einstellen, insbesondere eine definierte Temperatur und/oder definierte Luftfeuchte und/oder definierte Gaskonzentrationen, insbesondere eine definierte Kohlendioxid- und/oder Sauerstoffkonzentration, und/oder eine definierte Gaszusammensetzung. Darüber hinaus weist die erfindungsgemäße Produktionsanlage wenigstens eine Transporteinrichtung zum insbesondere kontinuierlichen Transport des Grundsubstrats innerhalb der Einhausung auf. Die erfindungsgemäße Produktionsanlage ist dabei insbesondere für eine kontinuierliche Herstellung des substratbasierten Myzelwerkstoffs konzipiert und ausgelegt.

Der Begriff "Wachstumsbedingungen" im Sinne der vorliegenden Erfindung ist insbesondere bezogen auf die Temperatur, den Feuchtegehalt und die Gaszusammensetzung bzw. die Gaskonzentrationen der Gasatmosphäre innerhalb der Einhausung.

Die Formulierung "vor einer Fruchtkörperbildung" im Sinne der vorliegenden Erfindung ist insbesondere zu verstehen als Zustand, in dem es auf und/oder an dem Substratmaterial zumindest im Wesentlichen noch nicht zu einer Fruchtkörperbildung des Pilzes gekommen ist. Damit unterscheidet sich das erfindungsgemäße Verfahren von den aus dem Stand der Technik bekannten Verfahren der Pilzkörperherstellung, bei denen die Verfahrensbedingungen, insbesondere Wachstumsdauern, auf die Pilzkörperbildung abgestimmt und damit länger sind als die Durchwachstumsdauer der Substratmaterials bei dem erfindungsgemäßen Verfahren.

Die Ausbildung von Fruchtkörpern bei Pilzen beruht häufig auf Signalen, wie eine drastische Veränderung der Umgebungstemperatur. Der Pilzstamm Trametes Versicolor beispielsweise benötigt zum Ausbilden von Fruchtkörperansätzen einen Abfall der Temperatur auf 6 °C bis 15 °C. Zur weiteren Fruchtkörperentwicklung bis hin zur Sporulation wird eine Temperatur von 10 °C bis 17 °C benötigt. Erfindungsgemäß erfolgt das Durchwachsen des Substratmaterials mit Pilzmyzel bei einer Myzel-Subtrat-Kultivierungstemperatur, die deutlich oberhalb der Fruktifikationstemperatur des jeweiligen Pilzes, das heißt oberhalb der Temperatur, bei der ein Pilz bevorzugt Fruchtkörper ausbildet bis hin zur Sporulation. Für den Pilzstamm Trametes Versicolor beispielsweise kann eine Myzel-Substrat-Kultivierungstemperatur konstant im Bereich oberhalb von 20 °C, insbesondere im Bereich zwischen 22 °C und 25 °C, gewählt werden, so dass keine Fruchtkörperausbildung und Sporulation befördert bzw. die Fruchtkörperausbildung und Sporulation gezielt unterdrückt wird.

Besonders bevorzugt ist erfindungsgemäß vorgesehen, die Fruchtkörperausbildung und/oder die Sporulation durch eine gezielte Einstellung der Myzel-Substrat-Kultivierungstemperatur, bei der das Durchwachsen des Substratmaterials mit Pilzmyzel in einer gegenüber der Umgebungsatmosphäre zumindest im Wesentlichen geschlossenen und/oder abtrennbaren Einhausung erfolgt, auf eine Temperatur festzulegen, die über der Fruktifikationstemperatur und/oder der sporulationsinduzierenden Temperatur / Sporulationstemperatur des eingesetzten Pilzes liegt.

Vorzugsweise liegt die Myzel-Substrat-Kultivierungstemperatur in einem Bereich von oberhalb 20 °C und/oder wenigstens 10 °C oberhalb der Fruktifikationstemperatur und/oder der sporulationsinduzierenden Temperatur des Pilzes.

Insbesondere kann die Konzentration, bzw. das Verhältnis von Sauerstoff und Kohlendioxid in der Umgebung, das Fruchtkörperwachstum beeinflussen. Eine erhöhte CO₂-Konzentration hemmt in der Regel das Fruchtkörperwachstum und fördert das Streckenwachstum, wenn bereits Ansätze vorhanden sind, während eine erhöhte Sauerstoffkonzentration das Ausbilden von Fruchtkörpern fördern kann. Erfindungsgemäß kann daher vorzugsweise das Durchwachsen des Substratmaterials mit Pilzmyzel in einer Umgebung mit erhöhten CO₂-Konzentrationen erfolgen, um Fruchtkörperwachstum und Sporulation jedenfalls zu hemmen, insbesondere zu verhindern. Die CO₂-Konzentration in der geschlossenen und/oder abtrennbaren Einhausung während einer Durchwachstumsphase des Substratmaterials kann in diesem Zusammenhang mehr als 500 ppm, insbesondere mehr als 600 ppm, weiter insbesondere mehr als 750 ppm und/oder bis zu 2.000 ppm betragen.

Die erfindungsgemäß hergestellten Myzelwerkstoffe zeichnen sich insbesondere durch eine homogene Verteilung der Hyphen im Substratmaterial und damit eine homogene Struktur der Myzelwerkstoffe und/oder insbesondere durch einen hohen Durchwachsungsgrad des Substratmaterials mit Pilzmyzel von wenigstens 50%, vorzugsweise von wenigstens 80%, weiter vorzugsweise von wenigstens 95% oder mehr, aus.

Beim Durchwachsen des Substratmaterials mit Pilzmyzel kommt es insbesondere zu einer Wechselwirkung zwischen dem Pilzmyzel und dem Substratmaterial, wobei sich das Pilzmyzel um das Substratmaterial legt und aus einer losen Substratschüttung, beispielsweise aus Stroh oder Holzstoffen, einen Werkstoffverbund schafft. Es findet eine Kontaktierung des Pilzmyzels mit dem Substratmaterial statt, wobei, vorzugsweise, Anteile des Substratmaterials von dem Pilz für den Aufbau seiner Biomasse verstoffwechselt werden. Der verstoffwechselte Anteil des Substratmaterials liegt typischerweise im Bereich von weniger als 1 % der Substratmasse.

Vorzugsweise ist der Begriff der "Durchwachsung" als ggfs. gänzlich von Myzel durchsetztes Grundsubstrat zu verstehen oder von Hyphen gänzlich erschlossenes Substrat. Ein Durchwachsungsgrad lässt sich beispielsweise mittels REM-Biegezugfestigkeit einer Stichprobe ermitteln.

Die erfindungsgemäße Produktionsanlage ist zur Durchführung des erfindungsgemäßen Verfahrens ausgebildet und weist eine gegenüber der Umgebung zumindest im Wesentlichen geschlossene und/oder abtrennbare Einhausung aus, wobei in der Einhausung definierte Wachstumsbedingungen, insbesondere eine definierte Temperatur und/oder definierte Luftfeuchte und/oder definierte Gaskonzentration und/oder Gaszusammensetzung, insbesondere eine definierte CO₂- und/oder O₂-Konzentration, für das Durchwachsen eines Substratmaterials als Grundsubstrat mit Pilzmyzel einstellbar sind, und wenigstens eine Transporteinrichtung zum insbesondere kontinuierlichen Transport des Substratmaterials innerhalb der Einhausung.

Das erfindungsgemäße Herstellungsverfahren und die erfindungsgemäße Produktionsanlage bieten eine Reihe von Vorteilen gegenüber konventionellen Batch-Prozessen zur Herstellung von substratbasierten Myzelwerkstoffen.

Erfindungsgemäß lassen sich substratbasierte Myzelwerkstoffe erstmals in Mengen bereitstellen, die einen industriellen Einsatz solcher Werkstoffe ermöglichen, beispielsweise im Größenbereich von 100.000 Tonnen durchwachsenem Substratmaterials als Myzelwerkstoff, der kontinuierlich aus der Einhausung ausgebracht wird. Insbesondere kann der Myzelwerkstoff als Endlosmaterial bereitgestellt werden oder der erzeugte Myzelwerkstoff wird beispielsweise als Sackware für eine Lagerung und/oder einen Transport bereitgestellt.

Insbesondere ist in einfacher Weise ein Scale-Up der Anlagentechnik möglich, verbunden mit der Möglichkeit einer weitgehenden Automatisierung des Herstellungsprozesses. Im Übrigen lässt die erfindungsgemäße Produktionsanlage eine schnelle, planbare und qualitätsgesicherte Produktion von substratbasierten Myzelwerkstoffen zu.

Ein hoher Automatisierungsgrad des erfindungsgemäßen Verfahrens kann insbesondere die Verwendung einer geeigneten Mess-, Steuerungs- und/oder Regelungstechnik vorsehen, um die Anzahl der händisch auszuführenden Arbeitsschritte bei der Myzelwerkstoffherstellung zu verringern, wobei insbesondere eine regelmäßige Kontrolle der Umgebungsbedingungen vorgesehen sein kann und/oder wobei insbesondere das Durchwachsen des Substratmaterials mit Pilzmyzel unter Verwendung von Sensoren überwacht und automatisiert gesteuert und/oder beendet werden kann.

Das erfindungsgemäße Herstellungsverfahren ist somit durch eine geringe Anzahl von händischen Arbeitsschritten geprägt und lässt eine kontrollierte Massenfertigung und eine Anwendung beispielsweise auch in der Bauindustrie zu.

Kosten der Herstellung können reduziert sowie eingesetztes Substratmaterial und eingesetzte Energie können effizienter genutzt werden, beispielsweise durch Abwärmenutzung. Ein hohes Abfallaufkommen durch üblicherweise in Batch-Prozessen eingesetzte Einweg-Gefäße oder Beutel entfällt.

Durch Ablauf der Durchwachstumsphase des eingesetzten Substratmaterials in der Einhausung der Produktionsanlage lassen sich in einfacher Weise definierte Umgebungs- bzw. Wachstumsbedingungen für das Durchwachsen des Substratmaterials mit Pilzmyzel sicherstellen. Die Einhausung kann ein Mikroklima mit gegenüber dem Umgebungsklima veränderter Temperatur und/oder veränderter Luftfeuchte und/oder veränderter Gaszusammensetzung, insbesondere CO₂- und/oder O₂-Konzentration, aufweisen.

Eine direkte Einwirkung der Wachstumsbedingungen im Inneren der Einhausung auf das Grundsubstrat während der Durchwachstumsphase fördert das Myzelwachstum. Die Herstellung von Myzelwerkstoffen in einer gegenüber der Umgebung zumindest im Wesentlichen geschlossenen und/oder abtrennbaren Einhausung bzw. einer Wachstumskammer als abgeschlossene Herstellungseinheit minimiert die Gefahr von Kontaminationen des Substratmaterials mit Fremdorganismen und reduziert Fehlchargen. Damit werden eine hohe Effizienz der Werkstoffherstellung und eine gleichbleibende Qualität des Materials sichergestellt.

Im Übrigen ist das Aufrechterhalten optimierter Klima- bzw. Wachstumsbedingungen in der Einhausung bei geringem Energieaufwand möglich, wobei die Größe der Einhausung in konstruktiv einfacher Weise an die gewünschte Produktionsmenge angepasst werden kann.

Für das Pilzwachstum sind unter anderem Kohlenhydratquellen, gelöster Stickstoff und Phosphor erforderlich. Zudem muss ausreichend Wasser vorhanden sein, so dass die Substrate von Myzel bewachsen und verstoffwechselt werden können.

Für das Wachstum von Pilzorganismen können unterschiedliche Substratmaterialien eingesetzt werden. Pilzorganismen können unterschiedliche Substrattypen und Kohlenstoffquellen für ihr Wachstum nutzen, die Eigenschaften des Myzelmaterials hängt hierbei von den jeweils verdauten Subtraten und deren Faserstruktur ab. Beispiele für Substratmaterialien, die erfindungsgemäß als Grundsubstrat für die Herstellung substratbasierter Myzelwerkstoffe eingesetzt werden können, sind Getreide, Stroh, insbesondere Weizenstroh oder Maisstroh, Weizenkleie, Bagasse, Papier, Holz, Sägemehl, Holzspäne sowie Woll-, Hanf- oder Seidenabfälle. Grundsätzlich können alle organischen und nicht toxischen Materialien, die insbesondere als landwirtschaftliche oder industrielle Abfälle anfallen, wie Fruchtschalen, ebenfalls als Grundsubstrate eingesetzt werden. Geeignete Substratmaterialien enthalten oft Zellulose, Lignin oder auch beides.

Die Bildung eines Inokulums kann mit einem Fadenpilz aus der Gruppe der Ascomyceten, Basidiomyceten, Deuteromyceten, Oomyceten und Zygomyceten durchgeführt werden. Das Verfahren wird vorzugsweise mit Pilzen aus der Abteilung der Basidiomyceten, insbesondere der Klasse der Holobasidiomyceten, durchgeführt.

Das Verfahren wird vorzugsweise mit einem Pilz durchgeführt, der ausgewählt ist aus der Gruppe bestehend aus:
- Pleurotus ostreatus
- Agrocybe brasiliensis
- Flammulina velutipes
- Hypholoma capnoides
- Hypholoma sublaterium
- Morchella angusticeps
- Macrolepiota procera
- Coprinus comatus
- Agaricus arvensis
- Ganoderma tsugae
- Inonotus obliquus
- Fomes fomentarius
- Fomitopsis pinicola
- Ganoderma lucidum
- Ganoderma applanatum
- Ganoderma pfeifferi
- Ganoderma adspersum, syn. Ganoderma australe sensu Ryvarden et Melo
- Ganoderma resinaceum
- Ganoderma carnosum
- Laetiporus sulphureus
- Trametes gibbosa
- Trametes versicolor

Aus der vorgenannten Auflistung konnten im Rahmen von Versuchen, die zur Erfindung geführt haben, insbesondere die nachfolgenden Pilze als besonders geeignet zur Herstellung von mit Pilzmyzel durchwachsenem Substratmaterial identifiziert werden:
- Ganoderma lucidum
- Ganoderma applanatum
- Ganoderma pfeifferi
- Ganoderma adspersum, syn. Ganoderma australe sensu Ryvarden et Melo
- Ganoderma resinaceum
- Ganoderma carnosum
- Trametes versicolor (besonders bevorzugt)

Zur kontinuierlichen Herstellung des Myzelwerkstoffs ist gemäß einem ersten Erfindungsaspekt ein kontinuierlicher Eintrag von Substratmaterials als Grundsubstrat in die Einhausung und/oder ein kontinuierlicher Austrag von durchwachsenem Substratmaterial als Myzelwerkstoff aus der Einhausung vorgesehen. Hierdurch wird eine ökonomische Massenproduktion des Myzelwerkstoffs ermöglicht.

Die erfindungsgemäße Produktionsanlage ist in diesem Zusammenhang zur kontinuierlichen Zuführung von Substratmaterial als Grundsubstrat in die Einhausung und/oder zur kontinuierlichen Abführung von Substratmaterial als Myzelwerkstoff aus der Einhausung ausgebildet und eingerichtet. Die erfindungsgemäße Produktionsanlage kann dazu eine Zuführeinrichtung zur insbesondere kontinuierlichen Zuführung von Grundsubstrat in die Einhausung und eine Abführeinrichtung zur insbesondere kontinuierlichen Abführung von Myzelwerkstoff aus der Einhausung aufweisen.

Nicht ausgeschlossen ist eine Ausführungsform, bei der das Substratmaterial als Grundsubstrat sequenziell in die Einhausung eingebracht wird, d.h. in vorgegebenen Chargen, oder bei der das durchwachsene Substratmaterial als Myzelwerkstoff sequenziell aus der Einhausung ausgebracht wird, d.h. in vorgegebenen Chargen. Vorzugsweise ist aber eine kontinuierliche Einbringung von Grundsubstrat in die Einhausung und eine kontinuierliche Ausbringung von Myzelwerkstoff aus der Einhausung vorgesehen.

Ein weiterer Aspekt der Erfindung sieht einen kontinuierlichen Materialtransport des Substratmaterials innerhalb der Herstellungslinie vor. Hierbei wird das Substratmaterial während der Verfahrensdauer von wenigstens einem Verfahrensschritt, weiter vorzugsweise der Verfahrensdauer von jedem Verfahrensschritt, und/oder von wenigstens einem Verfahrensschritt zu einem nachfolgenden Verfahrensschritt, insbesondere von jedem Verfahrensschritt zu einem jeweils nachfolgenden Verfahrensschritt, entlang der Herstellungslinie transportiert, insbesondere kontinuierlich.

Eine bevorzugte Ausführungsform des erfindungsgemäßen Verfahrens sieht vor, dass die Bereitstellung von Substratmaterial als Grundsubstrat, die Inokulation des Substratmaterials, das Durchwachsen des Substratmaterials und die Ausbringung des Substratmaterials als Myzelwerkstoff sowie, vorzugsweise, gegebenenfalls weitere Verfahrensschritte der Myzelwerkstoffherstellung, entlang einer kontinuierlichen Herstellungslinie erfolgen, in der das Substratmaterial die Verfahrensschritte nachfolgend durchläuft. Hierdurch wird eine ökonomische Massenproduktion des Myzelwerkstoffs möglich.

Die Durchwachstumsphase des Grundsubstrats erfolgt in der Einhausung der erfindungsgemäßen Produktionsanlage unter definierten, insbesondere gegenüber Umgebungsbedingungen, veränderten Wachstumsbedingungen, insbesondere bei definierter Temperatur und/oder definierter Luftfeuchte und/oder definierter Kohlendioxid- und/oder Sauerstoff-Konzentration der das Substratmaterial im Inneren der Einhausung umgebenden Gasatmosphäre.

Vorzugsweise sind die Wachstumsbedingungen in der Einhausung vom Einbringungsort des Substratmaterials als Grundsubstrats in die Einhausung bis zum Ausbringungsort des Substratmaterials als Myzelwerkstoff aus der Einhausung, also im Bereich einer Durchwachstumszone der Einhausung, einstellbar und zumindest im Wesentlichen konstant. Hierdurch wird eine hohe Effizienz beim Durchwachsen des Substratmaterials mit Pilzmyzel sichergestellt. Der Materialtransport des Substratmaterials innerhalb der Herstellungslinie erfolgt vorzugsweise unter definierten gleichen Wachstumsbedingungen in der Einhausung.

Im Übrigen kann eine Zonenbildung mit mehreren unterschiedlichen Klimazonen bzw. unterschiedlichen Wachstumsbedingungen innerhalb der Einhausung vorgesehen werden, so dass innerhalb der Einhausung unterschiedliche Klimazonen ausgebildet sind, durch die das Substratmaterial mit der Transporteinrichtung während der Durchwachstumsphase bewegt wird.

Insbesondere benachbart zum Einbringungsort des Substratmaterials in die Einhausung und/oder zum Ausbringungsort des durchwachsenen Substratmaterials aus der Einhausung kann es zu einer Vermischung der Gasatmosphäre im Inneren der Einhausung mit der die Einhausung umgebenden Gasatmosphäre kommen und damit zu einer nicht konstanten Ausbildung der Wachstumsbedingungen innerhalb der Einhausung zwischen dem Einbringungsort und dem Ausbringungsort. Eine Vermischung der Gasatmosphäre im Inneren der Einhausung mit der Umgebungsatmosphäre benachbart zum Einbringungsort und/oder benachbart zum Ausbringungsort ist somit nicht zwingend ausgeschlossen.

Um eine Durchmischung von Atmosphären bzw. Klimazonen mit unterschiedlicher Temperatur, Luftfeuchte und/oder Gaskonzentration, insbesondere CO₂- und/oder O₂-Konzentration, zu vermeiden, kann wenigstens eine Schleuse vorgesehen sein. Durch eine Schleuse kann insbesondere das Eindringen von Außenluft, Sporen, Keimen oder dergleichen in den Innenraum der Einhausung und damit in den Bereich des Substratmaterials verhindert werden. Eine geringfügige Vermischung der Gasatmosphäre im Inneren der Einhausung mit der Umgebungsatmosphäre benachbart zum Einbringungsort und/oder benachbart zum Ausbringungsort kann aber auch bei Verwendung einer Schleuse erfolgen und tolerierbar sein.

Die Schleuse kann aber auch zu einer Zonierung der Einhausung in mehrere Zonen mit unterschiedlichem Mikroklima und/oder unterschiedlichen Wachstumsbedingungen führen.

Um die Durchmischung von Atmosphären zwischen Mikroklimazonen innerhalb der Einhausung oder zwischen der Umgebungsatmosphäre und der Atmosphäre im Inneren der Einhausung, insbesondere angrenzend an den Einbringungsort des Substratmaterials als Grundsubstrat in die Einhausung und den Ausbringungsort des Substratmaterials als Myzelwerkstoff, zu vermeiden, kann durch eine Schleuse beispielsweise ein Luftschleier ausgebildet werden. Beispielsweise kann durch eine Schleuse ein Sperrluftstrom erzeugt werden, der auf einen schmalen Überhang trifft, der oberhalb von dem in der Einhausung transportierten Substratmaterial angeordnet und von dem Substratmaterial um beispielweise 5 bis 10 cm beabstandet ist. Ein Sperrluftstrom kann durch einen Filter, beispielsweise einen Hepafilter, geleitet werden, um Außenluft zu reinigen, Kontaminationen aus der Luft zu vermeiden und die Sterilität des Substratmaterials im Einbringungsbereich des Substratmaterials in die Einhausung zu erhalten. Eine ähnliche Abtrennung von Innenraum und Außenraum der Einhausung kann im Ausbringungsbereich der Einhausung vorgesehen sein, in dem der Myzelwerkstoff die Einhausung verlässt. Die Erzeugung von Sperrluftströmen mit und ohne Desinfektionsmöglichkeit ist möglich. Alternativ kann auch ein leichter Überdruck in der Einhausung erzeugt werden, so dass das Einströmen von Umgebungsluft in die Einhausung verhindert werden kann.

Die Schleuse kann auch wenigstens eine Lamelle und/oder einen Gummivorhang aufweisen, der den Gasübertritt zwischen zwei Mikroklimazonen und/oder dem Innenraumklima der Einhausung und dem Umgebungsklima erschwert.

Zwischen benachbarten Mikroklimazonen in der Einhausung können Teilbereiche bzw. Kammern der Einhausung mit kurzem Verweilraum vorgesehen sein, durch die das Substratmaterial hindurch transportiert wird. Das Substratmaterial kann dann beim Transport von einer Klimazone in eine benachbarte Klimazone Atmosphärenteile in eine zwischen den benachbarten Klimazonen ausgebildete Kammer mitführen. Aufgrund der Kammer zwischen zwei angrenzenden Mikroklimazonen kommt es nicht zur vollständigen Klimadurchmischung.

Optional kann eine Luftabsaugung im Übergangsbereich zwischen zwei angrenzenden Mikroklimazonen der Einhausung vorgesehen sein.

Eine weitere Möglichkeit zur Trennung von unterschiedlichen Mikroklimazonen der Einhausung und zur Verhinderung der Durchmischung von unterschiedlichen Mikroklimazonen können getrennt angetriebene Förderbänder vorgesehen sein, die durch ein Übergabesegment voneinander getrennt sind. Auf einem ersten Förderband transportiertes Substratmaterial kann dann über das Übergabesegment auf ein zweites Förderband übergeben werden. In diesem kurzen Übergabebereich kann eine physikalische und/oder atmosphärische Trennung realisiert werden.

Weiterhin sieht es das erfindungsgemäße Verfahren vorzugsweise vor, dass eine Inokulation des Substratmaterials und/oder auch eine Sterilisation des Substratmaterials innerhalb der Einhausung erfolgt. Hierdurch wird die kontinuierliche Herstellung des Myzelwerkstoffs weiter vereinfacht und optimiert. Zu diesem Zweck kann die Produktionsanlage eine Beimpfungseinrichtung zur Beimpfung des Substratmaterials mit einem Inokulum und/oder eine Sterilisationseinrichtung zur Sterilisation des Substratmaterials innerhalb der Einhausung aufweisen.

Eine Sterilisation des Substratmaterials kann vor der Innokulation innerhalb der Einhausung oder auch außerhalb der Einhausung in einer der Einhausung vorgelagerten Sterilisationseinheit erfolgen.

Zur Reinigung des Substratmaterials von möglichen Kontaminationen kann eine thermische Sterilisation beispielsweise durch Heißluftsterilisation bei trockener Hitze unter atmosphärischem Druck durchgeführt werden. Das Sterilgut wird für einige Stunden, beispielsweise zwei Stunden, einer Temperatur von beispielsweise weniger als 240 °C, vorzugsweise von weniger als 200 °C, insbesondere beispielsweise von 180 °C, ausgesetzt. Alternativ kann ein Autoklavieren bei Überdruck vorgesehen sein, beispielsweise im Temperaturbereich zwischen 100 und 140 °C, beispielsweise bei 120 °C, und einem Überdruck beispielsweise zwischen 2 und 5 bar, insbesondere zwischen 2 und 3 bar, in einem drucksicheren Raum mit Überdruckventil. Das Sterilgut kann dann je nach Beschaffenheit dem Überdruck und der Temperatur so lange ausgesetzt werden, bis eine Zieltemperatur an allen Punkten des Sterilgutes für einen vorgegebenen Zeitraum, beispielsweise von wenigstens 10 Minuten, insbesondere von wenigstens 20 Minuten, erreicht ist. Weiter ist eine thermische Sterilisation bei Normaldruck oder minimalem Überdruck möglich, insbesondere eine Pasteurisierung mit Dampf. Beispielsweise kann eine thermische Sterilisation mit heißem Wasserdampf oder Heißluft mit Feuchte bei einer Temperatur von beispielsweise 100 bis 140 °C für eine Zeitdauer von beispielsweise 30 bis 90 Minuten vorgesehen sein.

Weiter ist eine Sterilisation eines von dem Substratmaterial bildenden Schüttgutes bzw. der Partikel des Substratmaterials mit UV-Strahlung und/oder anderen Wellenlängen ionisierender Strahlung, beispielsweise mit Gamma-, Beta-, Elektronenund/oder Röntgenstrahlung, möglich. Das Schüttgut kann beispielsweise beim Abrieseln in die Einhausung, weiter beispielsweise durch einen schmalen Spalt, insbesondere von mehreren Seiten oder auch einseitig bestrahlt werden. Hierzu kann eine Sterilisationseinrichtung in die Einhausung integriert sein.

Weiterhin kann innerhalb der Einhausung eine Be- oder Entlüftung, insbesondere Gaszufuhr zum Substratmaterial, weiter vorzugsweise eine Begasung mit Kohlendioxid und/oder Sauerstoff, und/oder eine Nährstoffzugabe zum Substratmaterial erfolgen. Hierdurch lassen sich in einfacher Weise optimale Bedingungen für das Myzelwachstum einstellen.

Zu diesem Zweck kann die erfindungsgemäße Produktionsanlage wenigstens eine Be- und/oder Entlüftungseinrichtung, beispielsweise ein Gebläse, aufweisen. Insbesondere besteht die Möglichkeit, das Substratmaterial in der Einhausung direkt zu belüften, insbesondere während der Durchwachstumsphase, d.h. insbesondere nach der Innokulierung des Substratmaterials.

Während der Durchwachstumsphase kann eine ein- oder mehrfache Nährstoffzugabe zum Substratmaterial innerhalb der Einhausung vorgesehen sein. Für die Zufuhr von Nährstoffen, insbesondere von Mikronährstoffen, wie Stickstoff, Phosphor, Kalium, und/oder für die Zufuhr von Wachstumsbeschleunigern, wie Kohlenhydraten, zum Substratmaterial in der Einhausung kann wenigstens eine Dosiereinheit vorgesehen sein. Die Dosiereinheit sollte so ausgebildet sein, dass es durch die Zufuhr von Nährstoffen und/oder Wachstumsbeschleunigern im Wesentlichen zu keiner Änderung des Mikroklimas in der Einhausung durch zusammen mit den Nährstoffen und/oder Wachstumsbeschleunigern einströmende Umgebungsluft kommt.

Insbesondere während der Durchwachstumsphase kann eine Bestrahlung des Substratmaterials mit einer definierten Lichtquelle vorgesehen sein. Hierdurch lässt sich das Myzelwachstum positiv beeinflussen. Die erfindungsgemäße Produktionsanlage kann zu diesem Zweck eine Bestrahlungseinrichtung zur Bestrahlung des Substratmaterials in der Einhausung mit Licht definierter Wellenlänge und/oder Strahlungsintensität aufweisen.

Über die gesamte Einhausung oder in Teilbereichen kann das Grundsubstrat mit einem definierten Lichtspektrum (beispielsweise LED mit unterschiedlichen Rot-Blau-Grün-Anteilen sowie Infrarot) sowie definierter Lichtmenge (PPFD - Photosynthetic Photon Flux Density) beleuchtet werden. Hierdurch lassen sich in einfacher Weise optimale Bedingungen für das Myzelwachstum einstellen.

Vorzugsweise ist die Einhausung im Wesentlichen lichtdicht geschlossen und/oder schließbar. Durch Abtrennung des Innenraums der Einhausung von jeglichem äußeren Lichteinfluss lässt sich gegebenenfalls eine Fruchtkörperbildung des Pilzmaterials verhindern. Die Transporteinrichtung kann sich in einer vollständig lichtdichten Umgebung befinden. Das Myzelwachstum findet dann unter Lichtabschluss statt. Die Einhausung der Transporteinrichtung, insbesondere eines Förderbands der Transporteinrichtung, kann beispielsweise Sichtfenster und Wartungsklappen mit Schwarzdichtung umfassen. Damit ist der Innenraum der Einhausung von jeglichem äußeren Lichteinfluss abgetrennt und das Durchwachsen des Grundsubstrats kann unter Lichtabschluss erfolgen.

In der Einhausung kann auch eine Additivierung und Funktionalisierung des Substratmaterials erfolgen durch Zugabe von wenigstens einem Zusatz- oder Zuschlagstoff zum Substratmaterial, insbesondere durch Zugabe von wenigstens einem Imprägniermittel und/oder Flammschutzmittel und/oder Hydrophobiermittel und/oder Dämmmaterial. Hierdurch lässt sich in einfacher Weise Einfluss nehmen auf die Werkstoffeigenschaften des Myzelwerkstoffs. Zur Additivierung und/oder Funktionalisierung des Substratmaterials kann die Produktionsanlage eine Additivierungsund/oder Funktionalisierungseinheit aufweisen.

Nicht ausgeschlossen ist, dass eine Inokulation des Substratmaterials und/oder eine Sterilisation des Substratmaterials, eine Nährstoffzugabe zum Substratmaterial, oder auch eine Additivierung und Funktionalisierung des Substratmaterials, außerhalb der Einhausung in einem vor- oder nachgelagerten Verfahrensschritt erfolgen.

Die erfindungsgemäße Produktionsanlage weist wenigstens eine Transporteinrichtung, insbesondere aufweisend wenigstens ein Förderband oder eine insbesondere schienengeführte Transportstruktur auf, zum insbesondere kontinuierlichen Transport eines in die Einhausung eingebrachten Substratmaterials als Grundsubstrat innerhalb der Einhausung. Innerhalb der Einhausung ist ein insbesondere kontinuierlicher Transport des Substratmaterials vorgesehen, insbesondere wobei das Substratmaterial kontinuierlich entlang einer Transportstrecke von einem Einbringungsort des Substratmaterials als Grundsubstrat in die Einhausung zu einem Ausbringungsort des Substratmaterials als Myzelwerkstoff aus der Einhausung transportiert wird. Das Substratmaterial wird dann mit der Transporteinrichtung vorzugsweise kontinuierlich von der Bereitstellung, weiter vorzugsweise über den Verfahrensschritt Innokulation und gegebenenfalls den Verfahrensschritt Additivierung und Funktionalisierung, und während des Durchwachsens bis zur Bereitstellung von durchwachsenem Substratmaterial als Myzelwerkstoff von dem Einbringungsort bis zu dem Ausbringungsort transportiert. Der Materialtransport erfolgt innerhalb der Einhausung vorzugsweise während der gesamten Durchwachstumsphase des Substratmaterials.

Eine Aufbereitung des Substratmaterials als Grundsubstrat durch Zerkleinerung und/oder Homogenisierung und/oder Sterilisation und/oder Befeuchtung des Substratmaterials und/oder durch Zugabe von wenigstens einem von wenigstens einem vorbestimmten Pilz verstoffwechselbaren Nährmaterial zum Substratmaterial erfolgt vorzugsweite außerhalb der Einhausung, was die verfahrenstechnische Umsetzung erleichtert. Damit wird ein entsprechend aufbereitetes Substratmaterial bereitgestellt, welches für das nachfolgende Myzelwachstum in die Einhausung eingebracht werden kann. Die Aufbereitung kann aber auch zumindest teilweise innerhalb der Einhausung erfolgen, was insbesondere die Sterilisation des Substratmaterials betrifft.

Darüber hinaus ermöglicht die Myzelwerkstoffherstellung unter definierten Wachstumsbedingungen in einer Einhausung eine hohe Produktsicherheit und -qualität, da Bedingungen und Parameter der Werkstoffherstellung kontinuierlich überwacht und eingestellt werden können.

Zu diesem Zweck kann die Produktionsanlage eine Mess-, Steuerungs- und/oder Regelungseinrichtung zur Messung, Steuerung und/oder Regelung der Temperatur und/oder der Luftfeuchte und/oder der Gaszusammensetzung, insbesondere der Kohlendioxid- und/oder Sauerstoff-Konzentration, in der Einhausung aufweisen.

Zur Einstellung einer definierten Temperatur innerhalb der Einhausung kann die Produktionsanlage wenigstens eine Temperiereinrichtung aufweisen. Zur Messung der Temperatur in der Einhausung kann wenigstens ein Temperatursensor vorgesehen sein, so dass in einfacher Weise die Temperatur im Inneren der Klimakammer erfassbar und die Temperiereinrichtung in Abhängigkeit von den erfassten Messwerten steuerbar und/oder regelbar ist.

Zur Einstellung einer definierten Luftfeuchte in der Einhausung kann die Produktionsanlage wenigstens einen Feuchtesensor und eine steuer- und/oder regelbare Befeuchtungseinrichtung aufweisen.

Zur Messung und/oder Bestimmung der Gaskonzentration in der Einhausung kann wenigstens ein Gassensor, insbesondere ein CO₂- und/oder O₂-Sensor, vorgesehen sein. Ein CO₂- und/oder O₂-Gassensor kann die jeweilige Gaskonzentration ermitteln und die Gaskonzentration durch Zu- oder Abströmen des jeweiligen Gases in bzw. aus der Einhausung regulieren. Damit ist es in einfacher Weise möglich, optimale Bedingungen für das Myzelwachstum im Inneren der Einhausung einzustellen und durch Zuströmen oder Abströmen des jeweiligen Gases gezielt zu verändern. Beispielsweise kann die Transporteinrichtung Metalllanzen aufweisen, die in vorzugsweise gleichem Abstand oder nach einem vorgegebenen Lanzenraster angeordnet sind und die insbesondere auf mittlerer Schichthöhe des Grundsubstrats in das mit der Transporteinrichtung transportierte Grundsubstrat einstechen. Über die Lanzen ist eine insbesondere gleichbleibende oder auch angepasste Begasung des Grundsubstrats möglich, wobei die Begasung vorzugsweise im Bereich der mittleren Schichthöhe des Grundsubstrats beginnt und von dort das Grundsubstrat nach außen durchströmt.

Mit einer CO₂- und/oder O₂-Lanze ist eine gezielte Zuführung von Gas in das Substratmaterial möglich, was wiederum zu einer Förderung des Myzelwachstums führen kann. Beispielsweise kann vorgesehen sein, mehrere Metalllanzen seitlich und/oder von oben in das Substratmaterial einzuführen. Über die Metalllanzen wird eine entsprechende Gasatmosphäre in das Substratmaterial eingeführt. Dies kann in einem vorgegebenen Abstand von beispielsweise 30 cm und 50 cm über den gesamten Verlauf oder lediglich über einen Teil-Verlauf der Durchwachstumsstrecke des Grundsubstrats in der Einhausung vorgesehen sein.

Das Ende der Durchwachstumsphase ist insbesondere vor einer Fruchtkörperbildung des Pilzmaterials vorgesehen. Das erfindungsgemäße Verfahren kann in diesem Zusammenhang vorsehen, dass der Durchwachsungsgrad und/oder die Durchwachstumsdauer des Substratmaterials in der Einhausung gemessen, gesteuert und/oder geregelt wird und dass, vorzugsweise, durchwachsenes Substratmaterial als Myzelwerkstoff automatisch bei Erreichen eines definierten Durchwachsungsgrades und/oder einer definierten Durchwachsdauer aus der Einhausung ausgebracht wird.

Zur Bestimmung des Durchwachsungsgrads kann eine Kontrolleinheit zur Überwachung der Gasatmosphäre im Inneren der Einhausung vorgesehen sein. In Abhängigkeit von der Zusammensetzung der Gasatmosphäre, insbesondere der CO₂-und/oder O₂-Konzentration in der Innenluft der Einhausung, lässt sich der Durchwachsungsgrad insbesondere auf Grundlage von empirischen Daten ermitteln.

Alternativ kann zur Erkennung einer Myzelbildung an der Oberfläche des Substratmaterials eine Oberflächenanalyse des Substratmaterials durch bildgebende Methoden, beispielsweise unter Verwendung einer Spektralkamera und/oder einer RGB-Kamera, vorgesehen sein.

Alternativ kann eine Stichprobenentnahmeeinheit zur automatischen Entnahme von Stichproben aus dem Substratmaterial vorgesehen sein, um im Bereich der Transportstrecke des Substratmaterials im Inneren der Einhausung Materialproben aus dem Substratmaterial zu entnehmen und anhand der Materialproben den Durchwachsungsgrad zu ermitteln. Mit einer solchen Stichprobenentnahme lässt sich auch eine Qualitätssicherung des hergestellten Myzelwerkstoffs in einfacher Weise realisieren. Es ist zweckmäßig, wenn die Stichprobenentnahme zu keiner Änderung der Gaszusammensetzung im Inneren der Einhausung führt. Beispielsweise kann eine Probenentnahme über eine Probenentnahmelanze erfolgen, die von außen in die Einhausung geführt ist und eine Probenentnahme aus dem Grundsubstrat ohne Luftvermischung mit der Umgebungsluft zulässt.

Eine Qualitätskontrolle für Myzel-Substratmaterialien, die nach dem erfindungsgemäßen Verfahren hergestellt sind, kann auf verschiedene Weisen erfolgen.

Insbesondere kann eine insbesondere KI-geschützte Bildanalyse zur Ermittlung des Myzelwachstums und der Substratkontaktierung vorgesehen sein. Hier kann das Anlernen einer künstlichen Intelligenz zur Unterscheidung von Myzelfäden und Substrat und die Ermittlung der volumenbezogenen Myzelmenge sowie Kontaktierungsrate vorgesehen sein. Im Lichtmikroskop lässt sich das Myzelwachstum bei einer beispielhaft gewählten 200-fachen Vergrößerung erkennen, wobei Myzel als weiße, spinnennetzartige Fäden erkennbar sind, die das Substratmaterial durch- und umwachsen.

Weiter besteht die Möglichkeit zur Bewertung der Durchwachsung des Substratmaterials unter Verwendung von Halbzeug-Materialproben. Die mechanischen Kenndaten, beispielsweise die Druckfestigkeit, eines substrierten Myzelwerkstoffs beispielsweise verbessern sich mit zunehmender Durchwachsung. Dementsprechend kann beispielsweise für eine definierte Mischung eines Substrat-Myzel-Materials die Abhängigkeit der Druckfestigkeit von der Durchwachsungszeit ermittelt werden, wodurch Rückschlüsse auf den Durchwachsungsgrad möglich sind. Der Durchwachsungsgrad muss hierbei jedoch definiert werden, wobei beispielweise eine empirische (subjektive) Festlegung nach dem Erreichen einer bestimmten Druckfestigkeit herangezogen werden kann. Beispielsweise kann eine Druckfestigkeit von größer 0,10 MPa, insbesondere von 0,20 MPa oder darüber hinaus einem Durchwachsungsgrad von 100 % entsprechen.

Die Vorgehensweise bei der Bewertung der Durchwachsung unter Verwendung von Halbzeug-Materialproben kann wie folgt ablaufen: Zunächst werden zu unterschiedlichen Verweilzeiten bzw. Durchwachsungszeiten aus der Einhausung bzw. einem Reaktor Substrat-Myzel-Halbzeuge entnommen und zu Prüfkörpern hergestellt. Hier können beispielsweise Würfelproben mit einer Abmessung von beispielsweise 40 mm x 40 mm x 40 mm in einem Druckversuch nach DIN EN 1969-1 analysiert werden. Die erhaltenen Druckfestigkeiten werden anschließend in Abhängigkeit von der Durchwachsungszeit aufgetragen. Aus dem funktionalen Zusammenhang können entsprechende Zwischenwerte abgeleitet werden. Grundsätzlich steigt die Druckfestigkeit mit wachsender Durchwachsungszeit an.

Werkstoffe, die aus Substrat-Myzel-Halbzeugen hergestellt werden, und/oder Substrat-Myzel-Halbzeuge können folgende Kenndaten aufweisen:

| | |
|---|---|
| Dichte: | 20 bis 550 kgm⁻³ |
| Druckfestigkeit: | 0,02 bis 2,00 MPa |
| Wärmeleitfähigkeit: | 0,01 bis 0,75 Wm⁻¹K⁻¹ |

Die Mess-, Steuer- und/oder Regelungseinrichtung kann auch zur Messung, Steuerung und/oder Regelung der Transportgeschwindigkeit des Substratmaterials in der Einhausung ausgebildet sein.

Besonders bevorzugt können externe Stimuli zur Beschleunigung des Myzelwachstums vorgesehen werden. Zu den einsetzbaren Stimuli gehören unter anderem Vibration, Licht und Akustik.

Zur Beschleunigung des Myzelwachstums hat sich eine Schwingungserregung im Bereich von 50 bis 2.000 Hz, auch in Intervallen aufgeprägt, erwiesen.

Licht kann im gesamten sichtbaren Spektrum im Bereich von 360 nm bis 830 nm als Mischlicht eingesetzt werden, dazu insbesondere Nahinfrarot (NIR) bei 770 nm und Ultraviolett (UV) bei 200 bis 400 nm, auch in Intervallen aufgeprägt.

Eine Schallerzeugung kann im Frequenzbereich von 10 Hz bis 200 Hz, mit Spitzen bei 100 Hz, auch in Intervallen aufgeprägt, eingesetzt werden und zur Beschleunigung des Myzelwachstums führen.

Darüber hinaus kann innerhalb der Einhausung eine Schwingungserregung des Substratmaterials während der Durchwachstumsphase und/oder eine Schallerzeugung im Umgebungsbereich des Substratmaterials vorgesehen sein, um das Myzelwachstum zu fördern. Eine Schwingungserregung des Substratmaterials kann beispielsweise durch Schwingungsbewegungen der Transporteinrichtung, insbesondere eines Förderbands der Transporteinrichtung, realisiert werden. Insbesondere kann das Förderband durch ein Vibrationssystem, beispielsweise einen Vibrationstisch, in dem gesamten Prozess bzw. über die gesamte Transportlänge des Substratmaterials oder auch nur abschnittsweise in Schwingung versetzt werden. Zur Bewegung der Atmosphäre im Reaktor können Schallwellen, beispielsweise Ultraschall, Musik, Schallwellen mit definierter Schallfrequenz, in die Einhausung eingespeist oder dort erzeugt werden.

Die erfindungsgemäße Produktionsanlage kann zu diesem Zweck wenigstens eine Vibrationseinrichtung und/oder Beleuchtungseinrichtung und/oder wenigstens eine Schallerzeugungseinrichtung aufweisen.

Das erfindungsgemäße Verfahren kann einen Herstellungsprozess eines substratbasierten Myzelwerkstoffs bis hin zum fertigen Werkstück vorsehen.

Das erfindungsgemäße Verfahren umfasst insbesondere die Verfahrensschritte bis zur Ausbringung von durchwachsenem Substratmaterial als Myzelwerkstoff bzw. als Substrat-Myzel-Halbzeug aus der Einhausung, d.h. bis zum Ende einer ersten Wachstumsphase.

Mit dem nach dieser ersten Wachstumsphase erhaltenen Substrat-Myzel-Halbzeug können entsprechend, nach einer Formgebung, vorzugsweise anschließender weitergehender Wachstumsphase II und weiter vorzugsweise anschließender Trocknung, die zum Abtöten des Myzels führt, Werkstücke, wie Dämmplatten, Isolierungen und Verpackungen oder dergleichen, hergestellt werden.

Die Dichte und die Eigenschaften dieser Werkstücke hängen dabei insbesondere davon ab, wie stark das in der ersten Wachstumsphase erhaltene Substrat-Myzel-Halbzeug bei einer nachfolgenden Formgebung in einer Form verdichtet wird. Mit dem durchwachsenen Substratmaterial können dabei entsprechende Formkörper hergestellt werden, beispielsweise würfelförmige oder auch plattenförmige Formkörper. Hierzu ist vorzugsweise eine rein mechanische Verdichtung des durchwachsenen Substratmaterials vorgesehen, wobei ein einem Dämmmaterial ähnlicher Werkstoff halten werden kann.

Nach der mechanischen Verdichtung des durchwachsenen Substratmaterials in einer entsprechenden Form kann der so erhältliche Formkörper, insbesondere zusammen mit der Form, für eine vorgegebene Zeitdauer erneut in eine geeignet temperierte Umgebung, beispielsweise in einen Inkubator, eingebracht werden, was dazu führt, dass das Pilzmyzel, was durch den Formgebungsprozess bereichsweise auseinandergerissen und voneinander getrennt worden ist, wieder zusammenwachsen kann. Hierdurch wird eine hohe Stabilität des Formkörpers erhalten.

Anschließend kann das Myzel durch Trocknung des Formkörpers abgetötet werden.

Nach der Trocknung des Formkörpers, wenn der Pilz abgetötet ist, lässt sich der Formkörper thermisch mit einer Heizpresse verpressen, beispielsweise bei Temperaturen zwischen 120 °C und 250 °C. Durch die hohe Temperatur schmelzen beispielsweise ligninhaltige Komponenten, was zu einer verbesserten Festigkeit des Formkörpers führt. Die Temperatur und der Druck komprimieren den Formkörper und führen zu einer weiteren inneren Verklebung, so dass Pressspanplatten ähnliche Werkstoffe entstehen können.

Auch nachträgliche Imprägnierungen, Lackierungen oder das Aufbringen von mineralischen Schichten, beispielsweise Putz, auf den Formkörper sind möglich.

Nach der Bereitstellung des Myzelwerkstoffs kann dieser in wenigstens einem weiteren Verfahrensschritt vorzugsweise außerhalb der Einhausung einer Formgebung und/oder Zerkleinerung und/oder Erhitzung und/oder Imprägnierung unterzogen oder für den Transport und/oder eine Lagerung in ein Transport- und/oder Lagerbehältnis eingebracht werden. Die Produktionsanlage kann zu diesem Zweck wenigstens eine Presse und/oder Zerkleinerungseinrichtung und/oder eine Trocknungseinheit und/oder eine Imprägniereinheit für den Myzelwerkstoff und/oder eine Einrichtung zur Überführung des Myzelwerkstoffs in ein transport- und/oder lagerfähiges Behältnis aufweisen. Beispielsweise kann eine Absackung des hergestellten Myzelwerkstoffs vorgesehen sein, wenn dieser aus der Einhausung ausgebracht worden ist.

Auch kann eine Plattenfertigung aus dem Myzelwerkstoff ohne Verpressung erfolgen, wobei der Myzelwerkstoff mittels Fallmesser in Plattenstücke getrennt und diese dann durch Trocknung bei beispielsweise 30 °C bis 60 °C in ein stabiles Plattenmaterial überführt werden. Auch eine Verpressung des Myzelwerkstoffs ist möglich, wobei getrocknetes und/oder feuchtes Plattenmaterial mittels Hitze und Druck zu einer gepressten Myzelplatte auf die gewünschte Materialdichte verpresst werden kann, beispielsweise mit einer hydraulischen Heißdruckpresse.

Zum Imprägnieren des Myzelwerkstoffs, beispielsweise vorliegend als Plattenmaterial, kann das Material mittels Sprühdüsen mit vernebelbaren Naturstoffen und/oder Chemikalien ummantelt werden und wird dadurch funktionalisiert. Hierdurch lässt sich insbesondere ein ausreichender Brandschutz erreichen. Alternativ besteht die Möglichkeit, nach fertiger Durchsetzung des Substratmaterials mit Pilzmyzel, den Myzelwerkstoff durch eine Granulieranlage/Schneidmühle in kleine Teile zu zerkleinern und nachfolgend in Tüten abzusacken. Dieses Halbzeug kann als Handelsware dienen oder an anderen Produktionsstätten weiterverarbeitet werden.

Im Übrigen kann eine Trocknung des Myzelwerkstoffs vorgesehen sein, beispielsweise mit Heißluft bei einer Temperatur von 30 °C und 60 °C. Durch einen Feuchtesensor kann ermittelt werden, wann die in dem Material befindliche Feuchtigkeit gänzlich aus dem Material gedrungen ist. Eine Messung über Mikrowellenabsorption, eine kapazitive Messung oder eine Messung der elektrischen Leitfähigkeit ist ebenfalls möglich. Die erfindungsgemäße Produktionsanlage kann zu diesem Zweck wenigstens eine Trocknungseinrichtung aufweisen.

Die Erfindung wird nachfolgend anhand der Zeichnung exemplarisch beschrieben. In der Zeichnung zeigen
- Fig. 1: eine schematische Darstellung des Verfahrensablaufs eines erfindungsgemäßen Verfahrens zur kontinuierlichen Herstellung von mit Pilzmyzel durchwachsenem Substratmaterial als substratbasierter Myzelwerkstoff;
- Fig. 2: eine schematische Darstellung einer erfindungsgemäßen Anlage zur kontinuierlichen Herstellung von mit Pilzmyzel durchwachsenem Substratmaterial als substratbasierter Myzelwerkstoff in einer freigeschnittenen Seitenansicht;
- Fig. 3: die in Fig. 2 gezeigte Anlage in einer perspektivischen Ansicht schräg von oben und
- Fig. 4: eine schematische Darstellung einer Ausführungsform einer erfindungsgemäßen Anlage zur kontinuierlichen Herstellung von mit Pilzmyzel durchwachsenem Substratmaterial als substratbasierter Myzelwerkstoff mit einem konzeptionellen Ansatz für ein Scale-Up der Anlagentechnik und Herstellungskapazität.

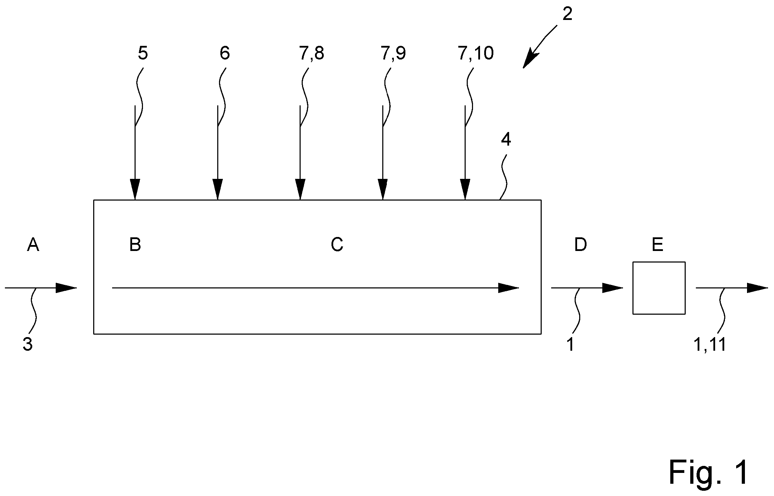

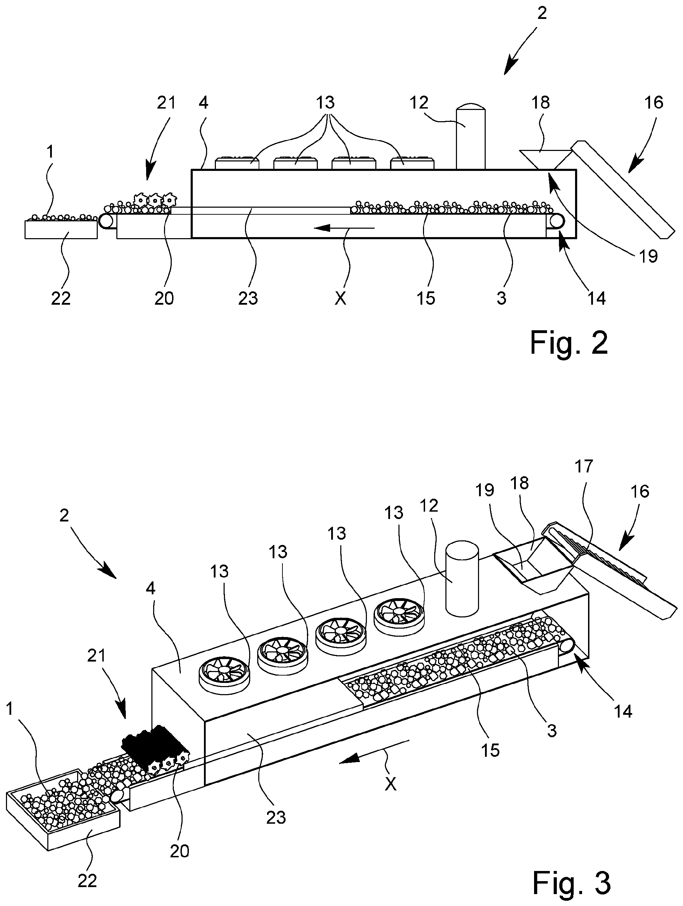

Fig. 1 zeigt schematisch den Verfahrensablauf eines Verfahrens zur kontinuierlichen Herstellung von mit Pilzmyzel durchwachsenem Substratmaterial als substratbasierter Myzelwerkstoff 1. Die Werkstoffherstellung erfolgt entlang einer Herstellungslinie bzw. Fertigungsstraße in einer Produktionsanlage 2, die in Fig. 1 lediglich als abstrahierte Anlagendarstellung und in den Fign. 2 bis 4 schematisch gezeigt ist.

Besonders bevorzugt sieht die kontinuierliche Herstellung des Myzelwerkstoffs 1 eine kontinuierliche Bereitstellung eines Substratmaterials als Grundsubstrat 3 für eine anschließende Inokulierung und eine kontinuierliche Bereitstellung von Myzelwerkstoff 1 vor.

In einem ersten Verfahrensschritt A erfolgt die Bereitstellung eines Substratmaterials als Grundsubstrat 3 für die Herstellung des Myzelwerkstoffs 1. Die Bereitstellung des Grundsubstrats 3 kann insbesondere eine Aufbereitung des Substratmaterials durch Zerkleinerung und/oder Homogenisierung und/oder Befeuchtung des Substratmaterials umfassen.

Anschließend wird das Grundsubstrat 3 in eine Einhausung 4 der Produktionsanlage 2 eingebracht. Die Einhausung 3 ist gegenüber der Umgebung zumindest im Wesentlichen geschlossen, wobei in der Einhausung 3 gegenüber der Umgebung veränderte Wachstumsbedingungen für ein optimiertes Myzelwachstum einstellbar sind.

Im zweiten Verfahrensschritt B erfolgt die Inokulierung des Grundsubstrats 3 durch Zugabe eines mit wenigstens einem vorbestimmten Pilz gebildeten Inokulums 5 zum Grundsubstrat 3. Bei dem Inokulum handelt es sich um Pilzmyzel oder Sporen wenigstens eines für das Durchwachsen des Grundsubstrats 3 mit Pilzmyzel bestimmten Pilzes.

Der zweite Verfahrensschritt B umfasst vorzugsweise auch eine vor der Inokulierung vorgesehene Sterilisation des Grundsubstrats 3, die aber auch ein Verfahrensschritt der Aufbereitung des Grundsubstrats 3 vor dem Einbringen in die Einhausung 4 sein kann.

In dem nächsten Verfahrensschritt C kommt es während einer Durchwachstumsphase des Grundsubstrats 3 im Inneren der Einhausung 4 zum wenigstens teilweisen, vorzugsweise vollständigen, Durchwachsen des Substratmaterials mit Pilzmyzel, wobei der Myzelwerkstoff 1 erhalten wird.

Während der Durchwachstumsphase kann gegebenenfalls eine Additivierung und Funktionalisierung des Grundsubstrats 3 durch Zugabe von wenigstens einem Zusatzstoff 6 bzw. Additiv zum Grundsubstrat 3, beispielsweise von wenigstens einem Imprägniermittel und/oder Flammschutzmittel und/oder Hydrophobiermittel und/oder Dämmmaterial.

Während der Durchwachstumsphase des Grundsubstrats 3 in der Einhausung 4 kann eine Messung, Steuerung und/oder Regelung insbesondere von Temperatur, Luftfeuchte und Gaszusammensetzung des Substratmaterials 3 im Inneren der Herstellungseinheit 2 unter Verwendung entsprechender Temperatur-, Feuchte- und Gassensoren vorgesehen sein, um optimale Wachstumsbedingungen für das Myzelwachstum einzustellen. Im Übrigen können eine nicht gezeigte Temperiereinrichtung und/oder eine Befeuchtungseinrichtung und/oder eine Gaszuführung zur Steuerung und/oder Regelung der vorgenannten Parameter vorgesehen sein.

Während der Durchwachstumsphase des Grundsubstrats 3 in der Einhausung 4 erfolgt vorzugsweise eine Belüftung des Grundsubtrats mit Umgebungsluft 7, wobei die Umgebungsluft 7 über Filter geleitet werden kann, um eine Kontamination des Grundsubstrats 3 in der Einhausung 4 mit Fremdorganismen zu unterbinden.

Durch Zufuhr eines Gasstroms 8, bestehend aus Kohlendioxid und/oder Sauerstoff, in die Einhausung 4 lässt sich die Gasatmosphäre in der Einhausung 4 für das Myzelwachstum optimieren.

Zudem besteht die Möglichkeit der Zufuhr von Nährstoffen 9 und/oder Wachstumsbeschleunigern 10 zum Grundsubstrat 3.

Vor einer Fruchtkörperbildung und nach Erreichen eines vorzugsweise vollständigen DurchwachsungsgradDurchwachsungsgrades des Grundsubstrats 3 mit Pilzmyzel ist das Ende der Durchwachstumsphase des Substratmaterials erreicht. Das durchwachsene Substratmaterial wird dann in einem Verfahrensschritt D als Myzelwerkstoff 1 aus der Einhausung 4 ausgebracht und außerhalb der Einhausung 4 bereitgestellt.

Der Myzelwerkstoff 1 kann anschließend in wenigstens einem weiteren Verfahrensschritt E be- und/oder weiterverarbeitet werden, wobei hier insbesondere eine Formgebung und/oder Erhitzung des Myzelwerkstoffs 1 vorgesehen sein kann. Durch Erhitzung wird ein weiteres Myzelwachstum unterbunden. Durch die Hitze wird zudem das Myzel zu einem Bioharz, wodurch ein sehr stabiler Werkstoff entsteht. Dies kann beispielsweise für eine Herstellung von stabilen Werkstücken 11 aus dem Myzelwerkstoff 1, wie beispielsweise Dämmplatten, vorgesehen sein.

Auch kann im Verfahrensschritt E eine Absackung des Myzelwerkstoffs 1 bzw. die Überführung des Myzelwerkstoffs 1 in Transportbehälter vorgesehen sein, um den Myzelwerkstoff 1 als lagerfähiges Zwischenprodukt bzw. Halbzeug für eine spätere Weiterverarbeitung, beispielsweise durch Formgebung und Finalisierung an anderen Produktionsstandorten, oder auch als Handelsprodukt bereitzustellen.

In den Fign. 2 und 3 ist eine Produktionsanlage 2 zur Herstellung von mit Pilzmyzel durchwachsenen Substratmaterial als substratbasierter Myzelwerkstoff 1 gezeigt. Insbesondere dient die Produktionsanlage 2 zur Herstellung von Myzelwerkstoff 1 nach dem oben beschriebenen Verfahren.

Die Produktionsanlage 2 weist eine gegenüber der Umgebung zumindest im Wesentlichen geschlossene und/oder abtrennbare Einhausung 4 auf. In der Einhausung 4 lassen sich definierte Wachstumsbedingungen, insbesondere eine definierte Temperatur und/oder definierte Luftfeuchte und/oder definierte CO₂- und/oder O₂-Konzentrationen, einstellen, so dass die Wachstumsbedingungen für das Durchwachsen eines Substratmaterials mit Pilzmyzel optimal einstellbar sind. Zudem wird durch die Einhausung 4 einer Kontamination des Substratmaterials während der Durchwachstumsphase mit Pilzmyzel vorgebeugt.

Die Inokulierung des Substratmaterials, das heißt die Zufuhr eines Inokulums 4 zum Grundsubstrat 3, erfolgt über eine schematisch gezeigte Beimpfungseinrichtung 12, die eine Beimpfung der Grundsubstrats vorzugsweise ohne Kontamination des Grundsubstrats 3 mit Fremdorganismen aus der Umgebungsluft der Einhausung 4 ermöglicht.

Nicht gezeigt ist, dass der Inokulierung eine Sterilisation des Substratmaterials vorausgehen kann. Zu diesem Zweck kann die Produktionsanlage 2 eine Einrichtung zur Sterilisation des Substratmaterials aufweisen, insbesondere integriert in die Einhausung 4. Beispielsweise kann eine Sterilisation über eine Bestrahlung des Grundsubstrats 3 innerhalb der Einhausung 4 vor der Inokulierung erfolgen. Eine Sterilisation des Substratmaterials ist aber auch vor der Einbringung in die Einhausung 4 möglich.

Zur Belüftung des Grundsubstrats 3 in der Einhausung 4 sind Belüftungseinrichtungen 13 vorgesehen. Zur Einstellung einer definierten Luftfeuchte lässt sich über die Belüftungseinrichtungen 13 ein Luftstrom gewünschter Feuchte dem Innenraum der Einhausung 4 zuführen. Nicht gezeigt ist, dass auch eine Befeuchtungseinrichtung zur Wasserzuführung zum Grundsubstrat 3 vorgesehen sein kann, beispielsweise über Lanzen.

Zur Einstellung einer definierten Temperatur in der Einhausung 4 kann wenigstens eine Temperiereinrichtung vorgesehen sein, die nicht gezeigt ist.

Die Einstellung einer definierten Gaskonzentration innerhalb der Einhausung 4 kann ein Kohlendioxid- oder Sauerstoffstrom in die Einhausung 4 eingeleitet werden. Die Gaszufuhr kann über nicht gezeigte Lanzen erfolgen, die den Gasstrom dem Grundsubstrat 3 zuführen. Eine direkte Begasung des Grundsubstrats 3 ist möglich. Beispielsweise können die Mündungsöffnungen der Lanzen in eine in der Einhausung 4 gebildete Schicht des Grundsubstrats 3 hineinreichen, um eine direkte Begasung des Grundsubstrats 3 zu realisieren.

Darüber hinaus weist die Produktionsanlage 2 eine Transporteinrichtung 14 mit einem Förderband 15 auf, um das Substratmaterial als Grundsubstrat 3 in der Einhausung 4 kontinuierlich vom Ort der Einbringung des Substratmaterials in die Einhausung 4 bis zum Ort des Austritts des durchwachsenen Substratmaterials als Myzelwerkstoff 1 aus der Einhausung in Transportrichtung X zu transportieren.

Um eine ausreichende Belüftung des Substratmaterials in der Einhausung 4 zu gewährleisten, können die Belüftungseinrichtungen 13 in Transportrichtung X nachfolgend angeordnet sein, so dass im Wesentlichen über die gesamte Transportstrecke des Substratmaterials in der Einhausung 4 eine Belüftung erfolgt.

Zum Einbringen des Substratmaterials als Grundsubstrats 3 in die Einhausung 4 ist eine Fördereinrichtung 16 mit einem weiteren Förderband 17 vorgesehen. Das Grundsubstrat 3 kann dann über einen Trichteraufbau 18 in die Einhausung 4 eintreten und gelangt von einem Einbringungsort 19 der Einhausung 4 vorzugsweise unter Schwerkrafteinfluss auf das innere Förderband 15. Von hier auf beginnt der kontinuierliche Transport des Grundsubstrats 3 in der Einhausung 4. Es schließen sich dann die oben beschriebenen Verfahrensschritte zur Herstellung von substratbasiertem Myzelwerkstoff 1 an. Der im Inneren der Einhausung 4 gebildete Myzelwerkstoff 1 wird dann mit dem Förderband 15 aus der Einhausung 4 abtransportiert und verlässt die Einhausung 4 an einem Ausbringungsort 20.

Vorzugsweise werden vom Einbringungsort 19 bis zum Ausbringungsort 20 im Wesentlichen gleiche Wachstumsbedingungen vorgegeben, was insbesondere die Temperatur und/oder die Feuchte und/oder die Gaszusammensetzung, weiter insbesondere die Kohlendioxid- und/oder Sauerstoffkonzentration, der Gasatmosphäre im Inneren der Einhausung 4 betrifft. Hierzu kann die Produktionsanlage 2 eine nicht gezeigte Temperiereinrichtung und/oder eine Befeuchtungseinrichtung aufweisen und/oder eine Gaszufuhr, insbesondere von Kohlendioxid und/oder Sauerstoff, mit einer Gaszufuhreinrichtung in die Einhausung 4 vorsehen.

Um eine Vermischung der Gasatmosphäre im Inneren der Einhausung 4 mit der Gasatmosphäre im Umgebungsbereich der Einhausung 4 durch Gasaustausch zwischen dem Innenbereich der Einhausung 4 und der Umgebung weitgehend zu reduzieren, kann ein nicht gezeigter Abschluss bzw. eine Schleuse vorgesehen sein. Schleusen zur Verhinderung einer Vermischung der Gasatmosphären können insbesondere im Bereich des Einbringungsorts 19 des Substratmaterials in die Einhausung und im Bereich des Ausbringungsorts 20 des durchwachsenen Substratmaterials aus der Einhausung 4 vorgesehen und ausgebildet sein.

Beispielsweise kann eine Schleuse durch einen Luftschleier bzw. einen Luftvorhang realisiert werden, um die Gasatmosphäre im Innenraum der Einhausung 4 von der Umgebungsatmosphäre im Bereich einer Öffnung der Einhausung 4 abzutrennen. Ein den Luftschleier bzw. Luftvorhang bildende Luftstrom kann über einen Filter geleitet werden, um den Luftstrom zu reinigen, Kontaminationen zu vermeiden und eine Sterilität des Grundsubstrats 3 für den nachfolgenden Herstellungsprozess des Myzelwerkstoffs 1 sicherzustellen.

In den Figuren 2 und 3 ist schematisch eine durchwachsene Substratschicht 23 gezeigt, die dem lockeren Grundsubstrat 3 ohne Bewuchs folgt. Es versteht sich, dass das Durchwachsen des Grundsubstrats 3 mit dem Pilzmaterial kontinuierlich auf dem Weg zum Ausbringungsort 20 erfolgt und die in den Figuren 2 und 3 gezeigte Grenze zwischen der durchwachsenen Substratschicht 23 und dem nicht durchwachsenen Grundsubstrat 3 lediglich beispielhaft und schematisch zu verstehen ist.

Dem Eintrag des Substratmaterials in die Einhausung 4 kann eine nicht gezeigte Einrichtung zur Aufbereitung des Substratmaterials, insbesondere durch Zerkleinerung und/oder Homogenisierung und/oder Befeuchtung und/oder Sterilisation und/oder Zugabe von einem von wenigstens einem vorbestimmten Pilz verstoffwechselbaren Nährstoff zum Substratmaterial, vorgeschaltet sein.

Nach der Inokulation können dem Substratmaterial auch Nährstoffe und/oder Wachstumsbeschleuniger zugeführt werden. Hierzu können entsprechend ausgebildete weitere Zuführeinrichtungen vorgesehen sein.

Darüber hinaus ist auch eine Zerkleinerung und/oder Homogenisierung des Myzelwerkstoffs 1 nach dessen Austrag aus der Einhausung 4 möglich. Hierzu kann eine Zerkleinerungseinrichtung 21 vorgesehen sein.

Der Myzelwerkstoff 1 kann dann in einem schematisch gezeigten Sammelbehälter 22 gesammelt und einer Weiterverarbeitung und/oder der weiteren Verwendung zugeführt werden. Auch eine Absackung des Myzelwerkstoffs 1 ist möglich.

Die Produktionsanlage kann eine nicht gezeigte Mess-, Steuer- und/oder Regelungseinrichtung aufweisen, die zur Messung, Steuerung und/oder Regelung der Temperatur und/oder der Luftfeuchte und/oder der Gaszusammensetzung in der Einhausung 4, insbesondere der Kohlendioxid- und/oder Sauerstoffkonzentration, ausgebildet sein kann.

Die Mess-, Steuerungs- und/oder Regelungseinrichtung kann auch zur Messung, Steuerung und/oder Regelung der Transportgeschwindigkeit des Förderbands 15 und damit der Verweilzeit des Substratmaterials in der Einhausung 4 und/oder zur Messung und/oder Steuerung und/oder Regelung der Durchwachsungszeit und/oder des DurchwachsungsgradDurchwachsungsgrads des Substratmaterials in der Einhausung 4 vorgesehen und ausgebildet sein. In Abhängigkeit von dem erreichten DurchwachsungsgradDurchwachsungsgrad kann die Transportgeschwindigkeit des Förderbands 15 gesteuert und/oder geregelt werden und damit die Verweilzeit des Substratmaterials in der Einhausung 4.

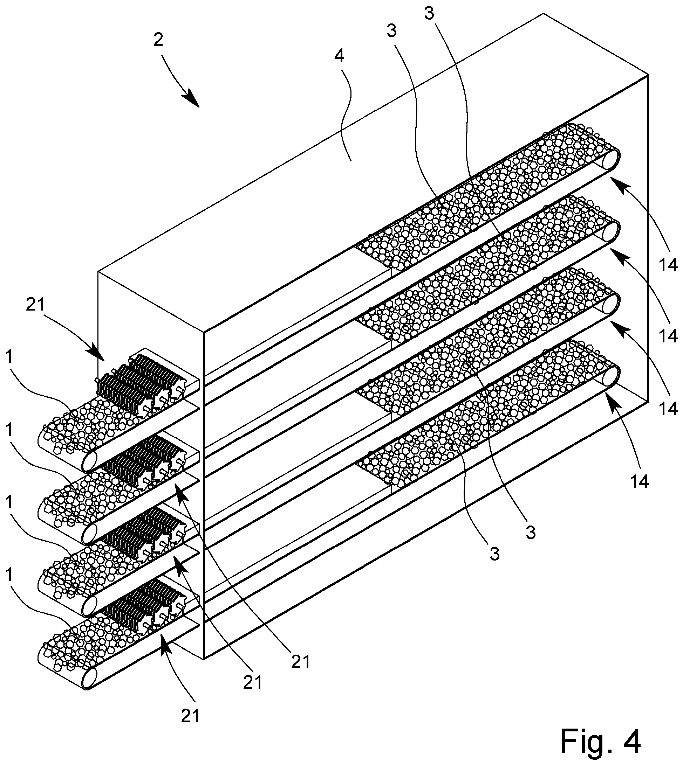

Fig. 4 zeigt schematisch die Möglichkeit für ein Scale-Up der Produktionsmenge von Myzelwerkstoff 1 unter Verwendung der Anlagentechnik der in den Fign. 2 und 3 gezeigten Produktionsanlage 2. Durch eine vertikale Anordnung mehrere Förderbänder 15 ist in einfacher Weise eine Steigerung der Produktionsmenge möglich.

Weitere Einrichtungen der in den Fign. 2 und 3 gezeigten Produktionsanlage 2, wie beispielsweise Einrichtungen zum Einbringen des Substratmaterials las Grundsubstrat 3 in die Einhausung 4, zum Austrag des durchwachsenen Substratmaterials als Myzelwerkstoff 1 aus der Einhausung 4, zur Inokulierung, Sterilisation, Belüftung des Substratmaterials sowie sonstige Einrichtungen, können in entsprechender Weise, gegebenenfalls auch mehrfach, bei der in Fig. 4 gezeigten Produktionsanlage 2 vorgesehen sein.

### Bezugszeichenliste:

- 1: Myzelwerkstoff
- 2: Produktionsanlage
- 3: Grundsubstrat
- 4: Einhausung
- 5: Inokulum
- 6: Zusatzstoff
- 7: Umgebungsluft
- 8: Gasstrom
- 9: Nährstoff
- 10: Wachstumsbeschleuniger
- 11: Werkstück
- 12: Beimpfungseinrichtung
- 13: Belüftungseinrichtung
- 14: Transporteinrichtung
- 15: Förderband
- 16: Fördereinrichtung
- 17: Förderband
- 18: Trichteraufbau
- 19: Einbringungsort
- 20: Ausbringungsort
- 21: Zerkleinerungseinrichtung
- 22: Sammelbehälter
- 23: Substratschicht

## Patentansprüche

1. Verfahren zur Herstellung von mit Pilzmyzel durchwachsenem Substratmaterial als substratbasierter Myzelwerkstoff (1), welches die Verfahrensschritte
- Bereitstellung eines Substratmaterials als Grundsubstrat (3) für die Herstellung von substratbasiertem Myzelwerkstoff (1) und, vorzugsweise, Aufbereitung des Substratmaterials durch Zerkleinerung und/oder Homogenisierung und/oder Befeuchtung und/oder Sterilisation und/oder durch Zugabe von wenigstens einem von wenigstens einem vorbestimmten Pilz verstoffwechselbaren Nährstoff zum Substratmaterial,
- Inokulation des Substratmaterials mit einem mit wenigstens einem vorbestimmten Pilz gebildeten Inokolum (5),
- Durchwachsen des Substratmaterials mit Pilzmyzel in einer gegenüber der Umgebungsatmosphäre zumindest im Wesentlichen geschlossenen und/oder abtrennbaren Einhausung (4) während einer Durchwachstumsphase des Substratmaterials zur Bildung von substratbasiertem Myzelwerkstoff (1) in der Einhausung (4) und
- Ausbringung von durchwachsenem Substratmaterial als Myzelwerkstoff (1) aus der Einhausung (4) vor einer Fruchtkörperbildung und, insbesondere, bei Erreichen eines definierten DurchwachsungsgradDurchwachsungsgrades und/oder einer definierten Durchwachstumsdauer des Substratmaterials in der Einhausung (4),
aufweist,
- wobei Substratmaterial kontinuierlich in die Einhausung (4) eingebracht und/oder durchwachsenes Substratmaterial kontinuierlich aus der Einhausung (4) ausgebracht wird und/oder
- wobei Substratmaterial innerhalb der Einhausung (4) kontinuierlich von einem Einbringungsort (19) des Substratmaterials als Grundsubstrat (3) in die Einhausung (4) zu einem Ausbringungsort (20) des Substratmaterials als Myzelwerkstoff (1) aus der Einhausung (4) transportiert wird.

2. Verfahren nach Anspruch 1, **dadurch gekennzeichnet, dass** die Bereitstellung von Substratmaterial als Grundsubstrat (3), die Inokulation des Substratmaterials, das Durchwachsen des Substratmaterials und die Ausbringung des Substratmaterials als Myzelwerkstoff (1) sowie, vorzugsweise, gegebenenfalls weitere Verfahrensschritte zur Herstellung von Myzelwerkstoff (1), entlang einer kontinuierlichen Herstellungslinie erfolgen, in der das Substratmaterial die Verfahrensschritte nachfolgend durchläuft.

3. Verfahren nach einem der vorhergehenden Ansprüche, **dadurch gekennzeichnet, dass** innerhalb der Einhausung (4) die Inokulation des Substratmaterials und/oder eine Sterilisation des Substratmaterials erfolgt.

4. Verfahren nach einem der vorhergehenden Ansprüche, **dadurch gekennzeichnet, dass** innerhalb der Einhausung (4) eine Be- oder Entlüftung, insbesondere Gaszufuhr, weiter vorzugsweise von Kohlendioxid und/oder Sauerstoff, zum Substratmaterial und/oder eine Nährstoffzugabe zum Substratmaterial und/oder eine Bestrahlung des Substratmaterials mit einer definierten Lichtquelle erfolgt.

5. Verfahren nach einem der vorhergehenden Ansprüche, **dadurch gekennzeichnet, dass** innerhalb der Einhausung (4) eine Additivierung und Funktionalisierung des Substratmaterials durch Zugabe von wenigstens einem Zusatzstoff (6), insbesondere von wenigstens einem Imprägniermittel und/oder Flammschutzmittel und/oder Hydrophobiermittel und/oder einem Dämmmaterial, zum Substratmaterial erfolgt.

6. Verfahren nach einem der vorhergehenden Ansprüche, **dadurch gekennzeichnet, dass** das Durchwachsen des Substratmaterials in der Einhausung (4) unter definierten, insbesondere gegenüber Umgebungsbedingungen, veränderten Wachstumsbedingungen erfolgt, insbesondere bei definierter Temperatur und/oder definierter Luftfeuchte und/oder definierter CO₂- und/oder O₂-Konzentration.

7. Verfahren nach einem der vorhergehenden Ansprüche, **dadurch gekennzeichnet, dass** der DurchwachsungsgradDurchwachsungsgrad und/oder die Durchwachstumsdauer des Substratmaterials in der Einhausung (4) gemessen, gesteuert und/oder geregelt wird und dass, vorzugsweise, durchwachsenes Substratmaterial als Myzelwerkstoff (1) automatisch bei Erreichen eines definierten DurchwachsungsgradDurchwachsungsgrades und/oder einer definierten Durchwachstumsdauer aus der Einhausung (4) ausgebracht wird.

8. Produktionsanlage (2) zur Herstellung von mit Pilzmyzel durchwachsenem Substratmaterial als substratbasierter Myzelwerkstoff (1) vor einer Fruchtkörperbildung, ausgebildet zur Durchführung eines Verfahrens nach einem der vorhergehenden Ansprüche, mit einer gegenüber der Umgebung zumindest im Wesentlichen geschlossenen und/oder abtrennbaren Einhausung (4), wobei in der Einhausung (4) definierte Wachstumsbedingungen, insbesondere eine definierte Temperatur und/oder definierte Luftfeuchte und/oder definierte Gaskonzentration und/oder Gaszusammensetzung, insbesondere eine definierte CO₂- und/oder O₂-Konzentration, für das Durchwachsen eines Substratmaterials als Grundsubstrat (2) mit Pilzmyzel einstellbar sind, und mit wenigstens einer Transporteinrichtung (14) zum insbesondere kontinuierlichen Transport des Substratmaterials innerhalb der Einhausung (4).

9. Produktionsanlage (2) nach Anspruch 8, **dadurch gekennzeichnet, dass** die Produktionsanlage (2) zur kontinuierlichen Zuführung von Substratmaterial als Grundsubstrat (3) in die Einhausung (4) und/oder zur kontinuierlichen Abführung von Substratmaterial als Myzelwerkstoff (1) aus der Einhausung (4) ausgebildet und eingerichtet ist.

10. Produktionsanlage (2) nach Anspruch 8 oder 9, **dadurch gekennzeichnet, dass** zumindest im Wesentlichen gleiche Wachstumsbedingungen für Myzelwachstum in der Einhausung (4) von einem Einbringungsort (19) des Substratmaterials als Grundsubstrat (3) in die Einhausung (4) bis zu einem Ausbringungsort (20) des Substratmaterials als Myzelwerkstoff (1) aus der Einhausung (4) einstellbar sind.

11. Produktionsanlage (2) nach einem der vorhergehenden Ansprüche, **dadurch gekennzeichnet, dass** eine Mess-, Steuerungs- und/oder Regelungseinrichtung zur Messung, Steuerung und/oder Regelung der Temperatur und/oder der Luftfeuchte und/oder der Gaszusammensetzung in der Einhausung (4), insbesondere der CO₂- und/oder O₂-Konzentration, und/oder zur Messung, Steuerung und/oder Regelung der Durchwachsungszeit und/oder des Durchwachsungsgrads des Substratmaterials in der Einhausung (4) vorgesehen ist, insbesondere wobei die Produktionsanlage (2) eine automatische Entnahme des Substratmaterials als Myzelwerkstoff (1) aus der Einhausung (4) bei Erreichen eines definierten Durchwachsungsgrads und/oder einer definierten Durchwachsungszeit vorsieht.
